# EUROPEAN PATENT APPLICATION

(11) **EP 0 569 233 A1**
(43) Date of publication of application: **10.11.1993**
(21) Application number: 93303501.6
(22) Date of filing: 05.05.1993
(51) Int. Cl.: A61B 19/02, A61B 17/32, A61M 5/32

(54) **Sharps handling apparatus**

(30) Priority: 05.05.1992 ZA 923225
(71) Applicant: HAUSLER SCIENTIFIC INSTRUMENTS (PTY) LIMITED, Johannesburg, Transvaal (ZA); Bongers, Dignus Eldert, Johannesburg, Transvaal (ZA)
(72) Inventor: Bongers Dignus, Eldert, Johannesburg, Transvaal (ZA)
(74) Representative: Harding, Richard Patrick

(57) **Abstract**

The sharps handling apparatus includes a blade removing device for removing a scalpel blade from a scalpel handle of the conventional type having a tang that retains the blade. The blade removing device (80) is located in a wall (26) which in the described embodiment is provided in the lid of a sharps container (12). There is an opening (82) in the wall and a ramp (94) which is inclined at an acute angle to the wall adjacent the opening. A slot (96) is formed in the ramp and extends down the incline and there is a detent (104) on the ramp. The arrangement is such that when the scalpel blade (90) is pressed into the opening in the wall, the blade retaining tang (92) can ride through the slot while the trailing end of the blade is obliged to ride along the operative underside of the ramp. This causes the blade to flex to a condition of disengagement with the tang. The blade is then engaged by the detent so that the scalpel handle can be separated from the blade. In the described embodiment, the blade then falls into the sharps container for safe disposal. The described embodiment also has the facility for removing hypodermic and other needles and for storing sutures in a safe and stick-free manner.

## Description

### BACKGROUND TO THE INVENTION

THIS invention relates to a sharps handling apparatus.

With the spread of blood transmitted diseases, notably AIDS and Hepatitis, a growing fear in the medical industry is that of accidental injury from needles or scalpel blades. Medical workers that remove needles from syringes or other apparatus after use, or that remove scalpel blades after invasive surgical procedures, are particularly prone to needle stick or scalpel blade cut or stab injuries and accordingly are especially at risk of contracting disease as a result.

With a view to reducing the risk of injury to such persons, there have already been a number of proposals to facilitate disposal of needles and scalpel blades without any necessity for hand contact with the needle or blade. For instance, in the case of scalpel blades, products sold under the trade names MARS-SAFETYBOX by Carl Melcher Medizin-Technik of Germany and SWANN-MORTON by Swann-Morton Limited of the UK are in the form of hinged receptacles which can be closed over the blade and manipulated to prise the blade off the scalpel handle. The handle is then withdrawn, leaving the blade enclosed in the receptacle which is then disposed of. A similar type of device is marketed under the trade mark FEATHER. Yet another similar device is described in US patent 5,088,173.

The disadvantage of these devices is the fact that they can be used only once and must then be disposed of along with the blade. This makes them rather expcnsive.

In the case of needles such as hypodermic or vacuum container needles, there exist proposals for high power incinerators that actually incinerate the needle in a short space of time. These devices are however expensive. PCT patent application PCT/US91/04084 describes a needle disposal apparatus in which an aperture in a disposal container is shaped to engage the hub of a threaded, double-ended needle thereby enabling the needle to he unscrewed and to fall through the aperture into the container. A somewhat similar arrangement is described in the specification of South African patent 91/0998. The specification of PCT/US92/07702 describes a disposal container having an aperture through which an entire hypodermic syringe can be pushed.

### SUMMARY OF THE INVENTION

The invention provides a sharps handling apparatus which includes a blade removing device for removing a scalpel blade from a scalpel handle having a tang that retains the blade, the device comprising a wall, an opening in the wall, a ramp which is inclined at an acute angle to the wall adjacent the opening, a slot formed in the ramp and extending down the incline, and a detent on the ramp, the arrangement being such that when the scalpel blade is pressed into the opening in the wall, the blade retaining tang rides along the slot while the trailing end of the blade rides along the ramp and the blade is flexed to a condition of disengagement with the tang, the blade then being engaged by the detent so that the scalpel handle can be separated from the blade when the handle is moved away from the opening.

The opening in the wall may be in the form of an elongate slot and preferably has a shape corresponding to the cross-section of a scalpel blade. The detent may comprise an undercut shoulder on the operative underside of the ramp behind which the trailing edge of the blade is engagable.

In the preferred embodiment, the wall also includes a first needle removing device adapted to remove slip-on type hypodermic needles from syringes. This device may comprise a key-hole shaped aperture formed in the wall and having larger and narrower parts, the larger part of the aperture being capable of receiving the hub of the slip-on type needle and the narrower part of the aperture being too narrow to allow passage of the hub.

The wall in the preferred embodiment also comprises a second needle removing device adapted to remove screw-on type needles from syringes or other apparatus. This device may comprise an aperture in the wall, slits formed through the wall and radiating from the aperture, the slits defining tapered, flexible fingers between them, the inner extremities of the fingers providing a locking dog to lock onto the hub of a needle inserted into the aperture and thereby to prevent rotation of the needle hub while unscrewing thereof takes place. The fingers are usually inclined relative to the wall.

Still further, the preferred embodiment comprises a plurality of membranes which are located in the wall and which are piercable by sutures. The membranes are conveniently formed by relatively thin sections of the wall, defined by tecesses in the wall. The recesses are preferably identified by different numbers.

There may be a container for accommodating removed sharps, the wall then forming part of a lid of the container and the blade removing device being arranged for a scalpel blade separated from a scalpel handle to fall into the container. The container and lid are typically each in the form of a one piece plastics moulding, the container and lid being mated with one another in clipping fashion. The apparatus may also include a cover engagable removably with the lid so as to extend over the lid, the cover being attached to the container and lid by a flexible linking member.

Another aspect of the invention provides a sharps handling device comprising a container for accommodating sharps and a lid structure including a wall which spans over the container, the wall including:
- a blade removing device adapted to remove a scalpel blade from a scalpel handle having a tang that retains the blade,
- a first needle removing device adapted to remove slip-on type hypodermic needles from syringes,
- a second needle removing device adapted to remove screw-on type needles from apparatus employing such needles, and
- a plurality of membranes which are thinner than the remainder of the wall and which are piercable by sutures,

the arrangement being such that a scalpel blade, a slip-on type needle or a screw-on type needle that has been removed by the relevant device is accommodated in the container.

### BRIEF DESCRIPTION OF THE DRAWINGS

The invention will now be described in more detail, by way of example only, with reference to the accompanying drawings in which:
- **Figure 1**: shows an exploded, perspective view of a sharps handling-apparatus of the invention;
- **Figure 2**: shows a side view of the same apparatus in an assembled condition with the cover closed;
- **Figure 3**: shows an enlarged plan view on the lid structure of the apparatus;
- **Figures 4A to 4C**: illustrate the operation of a device used to remove slip-on type needles from syringes;
- **Figure 5**: shows an enlarged cross-section through a device used to remove screw-on type needles;
- **Figure 6**: illustrates the operation of the device seen in Figure 5;
- **Figure 7**: illustrates the operation of a suture-receiving recess;
- **Figures 8 to 10**: show perspective views illustrating the operation of the scalpel blade removing device;
- **Figure 11**: shows a cross-section at the line 11-11 in Figure 8;
- **Figures 12 to 15**: show cross-sectional views illustrating the operation of the scalpel blade removing device; and
- **Figure 16**: shows a cross-section at the line 16-16 in Figure 14.

### DESCRIPTION OF AN EMBODIMENT

The illustrated sharps receptacle 10 has a lower container structure 12, a lid structure 14 and a cover 16. The container structure 12 is of moulded plastics material and is defined by a round cylindrical wall 18 and a base 20. The upper edge of the wall 18 is inwardly stepped and is formed with an undercut, peripheral rib 22

The lid structure 14 has a bounding, round cylindrical wall 24 with an undercut groove which receives the rib 22 of the wall 18 in clipping fashion when the lid structure is pressed downwardly onto the container structure. In addition, the lid structure 14 has a transversely spanning wall 26.

The cover 16 has a cover portion 28 with a finger-engagable tab 30 and a linking member 32. During the assembly of the lid structure 14 to the container structure 12, the free end of the linking member 32 is hooked over the upper edge of the wall 18 and the free end region of that member is then sandwiched between the walls 18 and 24 when the lid structure is clipped to the container structure.

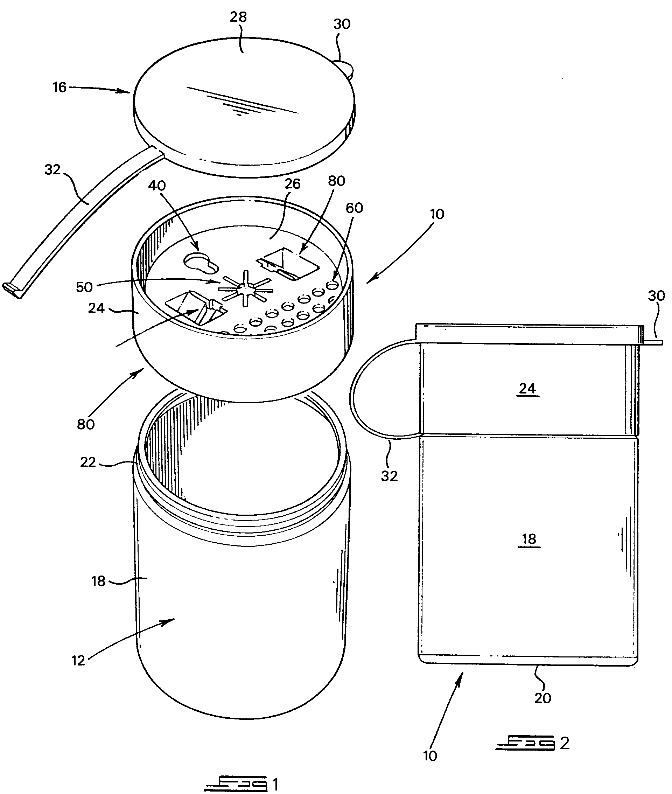

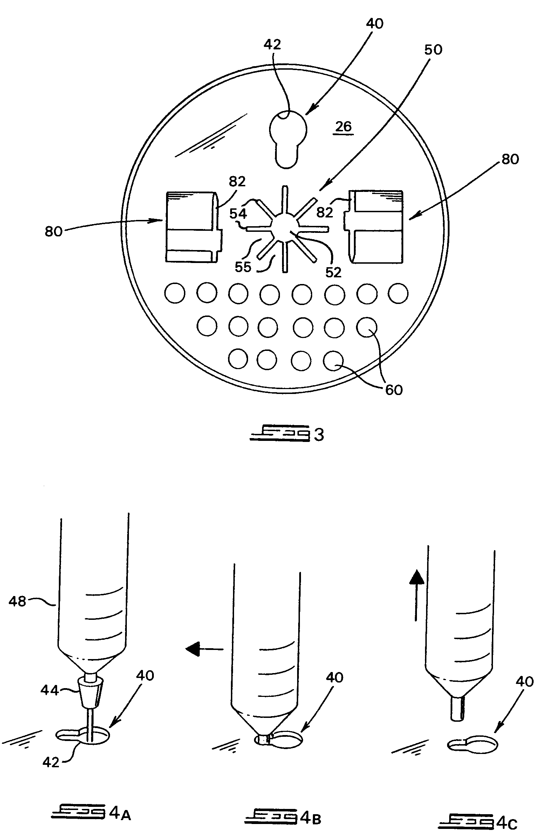

Figure 2 shows a view of the assembled receptacle, with the cover 16 in a closed position. When the receptacle is not in use, or is full of sharps and is to be disposed of, the cover portion 28 is merely swung **into** position over the upper edge of the wall 24 and is clipped thereto to cover the transverse wall 26, as illustrated.

It should be noted that when the lid structure is clipped onto the container structure, i.e. when the aforementioned undercut rib 22 of the container structure engages in the undercut groove of the lid structure, the lid structure cannot be detached from the container structure other than by application of a considerable deforming force. Thus when sharps such as needles or scalpel blades have been disposed of in the container structure, there is little danger of inadvertent finger contact with them.

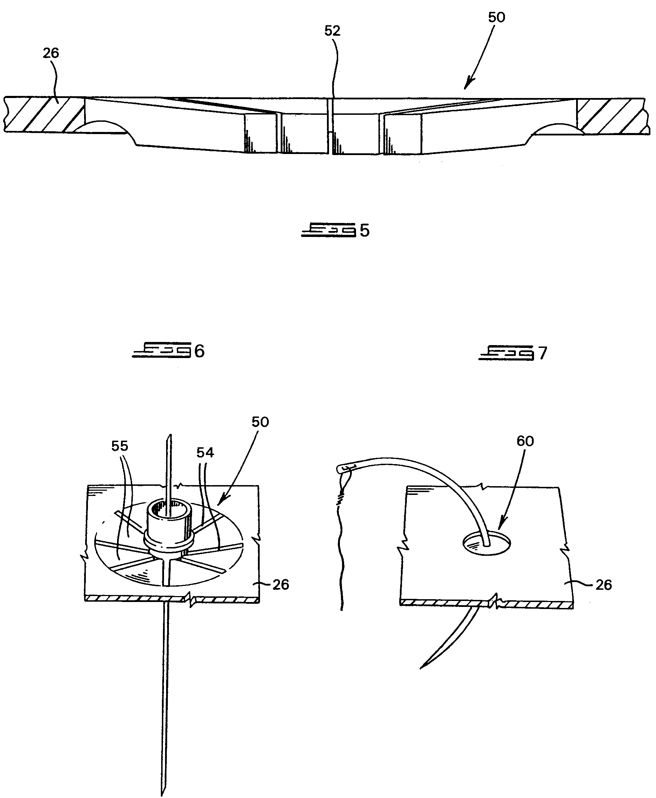

Referring specifically to Figure 3, the wall 26 includes a device 40 for removing conventional slip-on type hypodermic needles from syringes. This device consists of a key-hole shaped aperture 42 in the wall 26. In practice, the slip-on hub 44 of the needle 46 is inserted into the larger part of the key-hole aperture. The syringe 48 is then moved sideways to locate the hub of the needle beneath the narrower part of the key-hole aperture.

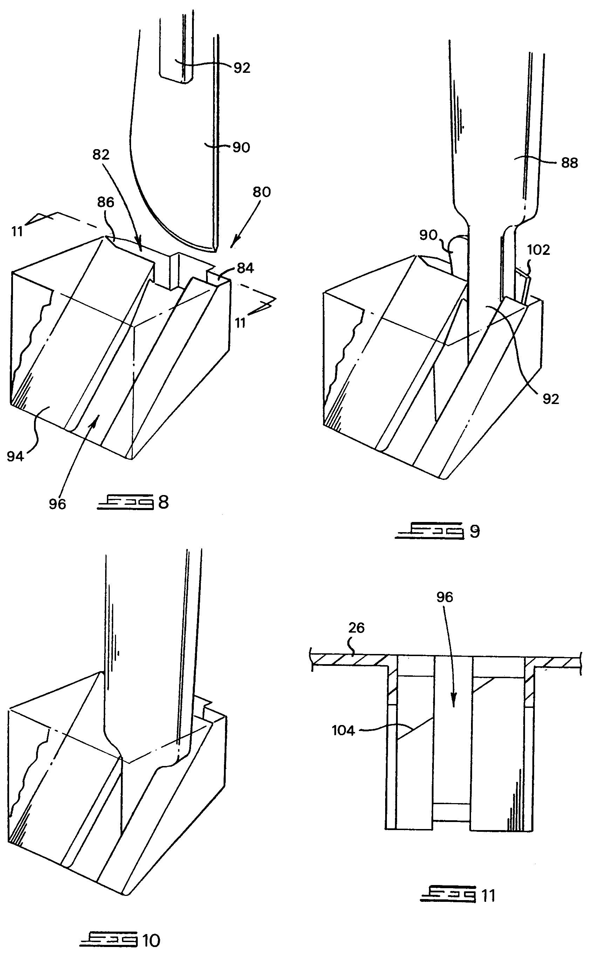

The syringe is now pulled away from the wall 26, or is tilted sideways, to disengage the needle hub from the syringe. The needle falls into the container structure 12 and is accordingly disposed of safely. This sequence of events is depicted in Figures 4A to 4C of the drawings.

The lid structure 14 also includes a device 50 for removing screw-on type needles, such as screw-on type needles used with syringes and double-ended needles as used in vacuum container applications.

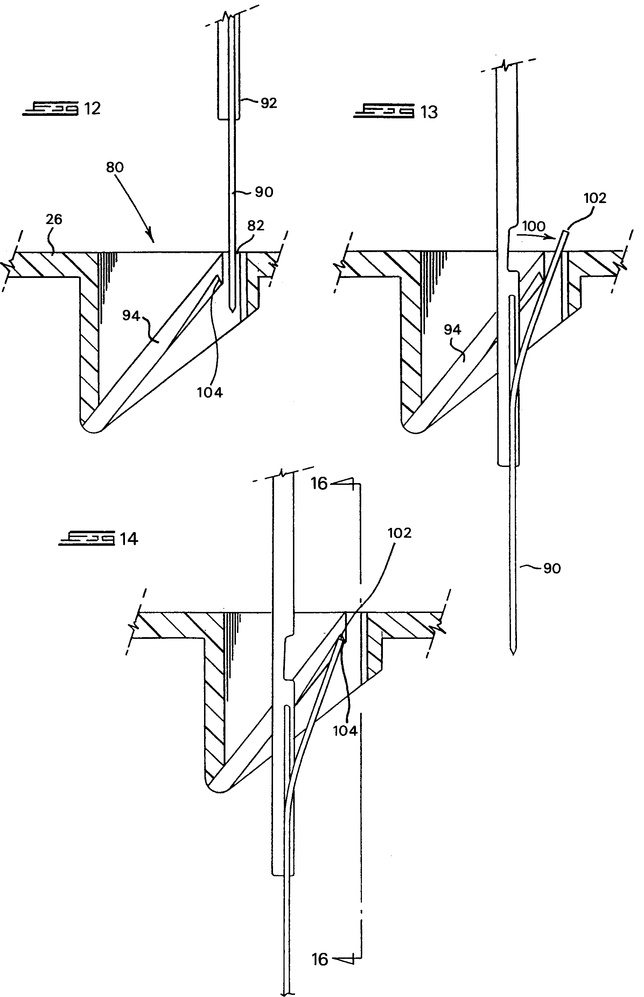

The device 50 is provided by a locking dog having a central aperture 52 and a series of slits 54 extending outwardly from the central aperture and defining a series of elements or fingers 55 of tapered shape. As illustrated in the cross-sectional view of Figure 5, the tapered fingers 55 are not coplanar with the remainder of the wall 26, but slope downwardly, i.e. into the container structure 12, at a small inclination towards the central aperture.

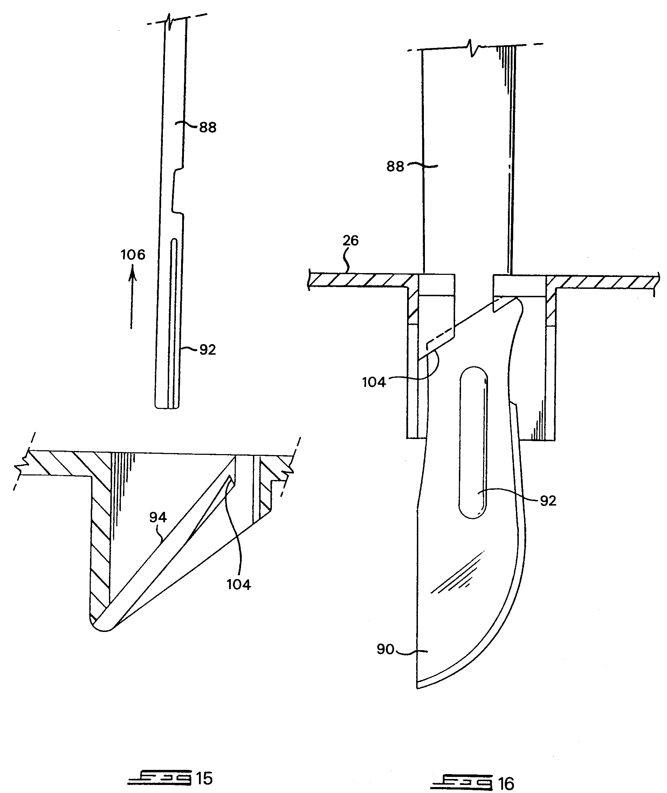

In operation, the needle which is to be removed and disposed of is inserted through the aperture 52. In Figure 6, which illustrates the fully inserted needle, the syringe or other device to which the needle is screwed is omitted. The needle is pressed through the aperture 52 until such time as the hub at the base of the needle is lodged firmly in the aperture. This pressing movement of the needle through the aperture is accompanied by downward flexure of the tapered fingers 55.

Common to screw-on type needles is a hub which has an out-of-round shape. Typically, the hub has a series of circumferentially spaced ribs or vanes. The out-of-round nature of the hub results in its being trapped non-rotatably in the aperture 52.

In some cases, it may be the inner extremities of the fingers which grip the hub, while in other cases, vanes on the hub may be caught in the slits 54. Once the hub has been captured firmly in the aperture, the syringe or other device to which the needle is attached is unscrewed from the needle merely by applying a rotary action to it while holding the receptacle 10 firm against rotation.

Once the needle has been completely unscrewed, the operator applies further downward force to the syringe or other device to force the hub of the needle right through the aperture, and allowing the detached needle to drop into the container structure 12. It will be appreciated that this movement of the needle is accommodated by further downward deformation of the fingers 55 to allow passage of the needle hub. It will also be appreciated that the lid structure 14, or at least the wall 26 thereof, must have sufficient resilience to permit the require flexure of the fingers 55.

It is to be noted that once the fingers 55 have been flexed downwardly to accept the needle hub, it is not possible to pull the needle hub out of the aperture 52 because the inclination and flexure of the fingers is such that they lock onto the hub. This anchors the hub against rotation during unscrewing. Once the needle has dropped into the container structure 12, it no longer presents a danger and cannot be retrieved.

The wall 26 also includes a series of recesses 60 which extend partially through the thickness of the wall The base of each recess is defined by a thin membrane of plastics material. The recesses 60 are used to receive sutures or other slender sharps.

In practice, when a suturing operation has been completed, the point of the used suture is pressed through the thin membrane. The suture is only partially driven through the membrane and its upper end is left visibly protruding, as illustrated in Figure 7. During this suture storage procedure, there is little danger of finger contact with the sharp end of the suture.

Each of the recesses 60 is numbered, typically by means of a raised number formed in the plastics material during moulding thereof. Sutures will be anchored in numerical sequence in the recesses so that, at the end of a surgical procedure, it is possible to see exactly how many sutures have been retrieved and stored. In the illustrated case, it is possible to store up to 18 sutures.

The recesses make it a simple matter to avoid inadvertent loss of a suture in a wound in a patient's body. In practice, the upper edge of the wall 24 is elevated far enough above the wall 26 to allow the cover 16 to close properly over the protruding ends of the sutures.

An important feature of the illustrated receptacle 10 is the provision of devices 80 to remove scalpel blades safely and without contact by the human hand. In this embodiment, there are two identical devices 80. The operation of one of these devices is described in detail below with reference to Figures 8 to 16.

Referring to Figure 8, each device 80 includes a slot 82 in the wall 26. The slot has a shape corresponding to the cross-section of a typical scalpel blade, with a rectangular end 84 and a pointed end 86. Figure 8 also shows the leading end of a no. 4 scalpel 88 fitted with a no. 4 blade 90. The tang of the scalpel 88 over which the blade clips in conventional manner is indicated with the numeral 92. Leading from the underside of the wall 26, at an acute angle to that underside, is an inclined ramp 94 formed with a slot 96 that communicates with the slot 82.

In practice, the blade is inserted into the slot 82 as illustrated in Figures 8 and 12. The shape of the slot 82 only permits the blade to enter in the correct manner rather than back to front. The scalpel is pressed downwardly so that the tang 92 enters and rides down the slot 96. The blade 90 is too broad to enter the slot 96 and rides down the underside of the ramp 94 as seen in Figure 13. As will be appreciated from a reading of Figures 9, 10 and 13, the action described flexes the blade in the direction of the arrow 100 and disengages it from the tang.

The rear edge 102 of the blade' moves, with a clipping action, past an undercut shoulder 104 on the underside of the ramp 94, as illustrated in Figure 14. As will be clear from Figure 16, the shoulder 104 has an inclination corresponding to that of the standard rear edge 102 of the blade 90.

When the operator detects that the edge 102 of the blade has clipped past the shoulder 104, he applies a pulling action to the scalpel handle as illustrated by the arrow 106 in Figure 15. The blade, which is arrested by the shoulder, slips off the tang 92 and drops into the container 12.

It will be noted that the entire' blade removal operation is conducted without any finger contact with the blade and that, at the end of the operation, the blade is stored safely in the container structure. The provision of two blade removal devices 80 in the receptacle 10 will, it is believed, facilitate blade removal procedures and obviate the need for excessive rotation of the receptacle 10 to align the slots 82 at appropriate positions for comfortable operation.

Although reference has been made to a no. 4 scalpel and blade it is to be recognised that other blades can be removed from other standard scalpel handles using the devices 80.

It is believed that the receptacle 10 as described above will be extremely useful, particularly bearing in mind that it has the facility for safely disposing of a wide variety of sharps used in the medical industry. When it is desired to dispose of the sharps in the container structure 12, the whole receptacle 10 can be incinerated.

## Claims

1. A sharps handling apparatus which includes a blade removing device for removing a scalpel blade from a scalpel handle having a tang that retains the blade, characterised in that the device (80) comprises a wall (26), an opening (82) in the wall, a ramp (94) which is inclined at an acute angle to the wall adjacent the opening, a slot (96) formed in the ramp and extending down the incline, and a detent (104) on the ramp, the arrangement being such that,when the scalpel blade (90) is pressed into the opening in the wall, the blade retaining tang (92) rides along the slot while the trailing end of the blade rides along the ramp and the blade is flexed to a condition of disengagement with the tang, the blade then being engaged by the detent so that the scalpel handle (88) can be separated from the blade when the handle is moved away from the opening.

2. A sharps handling apparatus according to claim 1 characterised in that the opening (82) in the wall is in the form of an elongate slot.

3. A sharps handling apparatus according to claim 2 characterised in that the elongate slot (82) has a shape corresponding to the cross-section of a scalpel blade (90).

4. A sharps handling apparatus according to any one of the preceding claims characterised in that the detent (104) comprises an undercut shoulder on the operative underside of the ramp (94) behind which the trailing edge (102) of the blade (90) is engagable.

5. A sharps handling apparatus according to any one of the preceding claims characterised in that the wall (26) also includes a first needle removing device (40) adapted to remove slip-on type hypodermic needles from syringes (48).

6. A sharps handling apparatus according to claim 5 characterised in that the first needle removing device (40) comprises a key-hole shaped aperture (42) formed in the wall (26) and having larger and narrower parts, the larger part of the aperture being capable of receiving the hub (44) of the slip-on type needle and the narrower part of the aperture being too narrow to allow passage of the hub.

7. A sharps handling apparatus according to any one of the preceding claims characterised in that the wall (26) comprises a second needle removing device (50) adapted to remove screw-on type needles from syringes or other apparatus.

8. A sharps handling apparatus according to claim 7 characterised in that the second needle removing device 50) comprises an aperture (52) in the wall (26), slits (54) formed through the wall and radiating from the aperture, the slits defining tapered, flexible fingers (55) between them, the inner extremities of the fingers providing a locking dog to lock onto the hub of a needle inserted into the aperture and thereby to prevent rotation of the needle hub while unscrewing thereof takes place.

9. A sharps handling apparatus according to claim 8 characterised in that the fingers (55) are inclined relative to the wall (26).

10. A sharps handling apparatus according to any one of the preceding claims characterised in that the wall (26) comprises a plurality of membranes which are located in the wall and which are piercable by sutures.

11. A sharps handling apparatus according to claim 10 characterised in that the membranes are defined by relatively thin sections of the wall (26), defined by recesses (60) in the wall.

12. A sharps handling apparatus according to claim 11 characterised in that the recesses (60) are identifiedl by different numbers.

13. A sharps handling apparatus according to any one of the preceding claims characterised by a container (12) for accommodating removed sharps, the wall (26) forming part of a lid (14) of the container and the blade removing device (80) being arranged for a scalpel blade (90) separated from a scalpel handle (88) to fall into the container.

14. A sharps handling apparatus according to claim 13 characterised in that the container (12) and lid (14) are each in the form of a one piece plastics moulding, the container and lid being mated with one another in clipping fashion.

15. A sharps handling apparatus according to claim 14 characterised by a cover (16) engagable removably with the lid (14) so as to extend over the lid, the cover being attached to the container and lid by a flexible linking member (32).

16. A sharps handling device comprising a container for accommodating sharps and a lid structure including a wall which spans over the container, characterised in that the wall (26) includes the combination of:
- a blade removing device (80) adapted to remove a scalpel blade (90) from a scalpel handle (88) having a tang (92) that retains the blade,
- a first needle removing device (40) adapted to remove slip-on type hypodermic needles from syringes (48),
- a second needle removing device (50) adapted to remove screw-on type needles from apparatus employing such needles, and
- a plurality of membranes which are thinner than the remainder of the wall and which are piercable by sutures,
and further characterised in that the arrangement is such that a scalpel blade (90), a slip-on type needle or a screw-on type needle that has been removed by the relevant device (40, 50, 80) is received by the container.

17. A sharps handling apparatus according to claim 16 characterised in that the blade removing device (80) includes means (94, 96) operable to flex the scalpel blade (90) to disengage it from the tang (92) and means (104) to detain the disengaged blade to enable the scalpel handle (88) to be separated from the blade.
